# FASCICULE DE BREVET EUROPEEN

(11) **EP 1 911 473 B1**
(45) Date de publication et mention de la délivrance du brevet: **16.07.2014**
(21) Numéro de dépôt: 07020099.3
(22) Date de dépôt: 02.03.2005
(51) Int. Cl.: A61M 1/00, A61M 3/02

(54) **Machine d'irrigation ou d'aspiration utilisée en endoscope**
Spül- und Saugmaschine, die in einem Endoskop benutzt wird
Irrigation or suction machine used in endoscopy

(30) Priorité: 05.03.2004 EP 04100917; 18.06.2004 FR 0406620
(43) Date de publication de la demande: 16.04.2008
(62) Demande divisionnaire de: 05706542.7
(73) Titulaire: Future Medical System S.A., 2400 Le Locle (CH)
(72) Inventeur: Pascual, Thierry, 06700 Saint Laurent du Var (FR); Chautard, Stanislas, 92200 Neuilly sur Seine (FR); Dias, Armando, 06300 Nice (FR); Tachoire, Raphael, 06700 Saint Laurent du Var (FR); Janin, Patrick, 06000 Nice (FR); Janin, Steven, 06200 Nice (FR); Francisco, André, 06640 St. Jeannet, Nice (FR); Rodriguez, Christian, 06640 St. Jeannet, Nice (FR)
(74) Mandataire: Moinas & Savoye SA

(56) Documents cités:
- US-A- 5 246 422
- US-A- 5 460 490
- US-A- 5 626 563
- US-A- 5 628 731
- US-A- 5 649 905
- US-A1- 2002 147 423
- US-A1- 2003 229 302

## Description

### Domaine technique

L'invention se rapporte à une machine d'irrigation ou d'aspiration utilisée en endoscopie, selon le préambule de la revendication 1.

### Etat de la technique

Une machine de ce type est connue du document US 2003/229302.

Le but de l'invention est de rendre une telle machine plus simple à utiliser en autorisant le chargement de la cassette dans la machine.

### Divulgation de l'invention

A cet effet, l'invention a pour objet une machine selon la revendication 1.

Une telle machine est destinée à recevoir une cassette comprenant une tubulure d'irrigation ou une tubulure d'aspiration et un support muni d'une ou de deux prises d'entrée et d'une ou de deux prises de sortie, la ou les deux tubulures formant un coude pour s'engager avec la ou les deux prises d'entrée et de sortie suivant un sens de circulation respectivement entrant et sortant et formant un segment de pompage d'irrigation ou d'aspiration suivant le sens entrant de circulation.

Le support comprend un guide en T conformé à la tête du T pour protéger le coude de chaque tubulure et conformé le long du corps du T en un logement guidant la ou les deux tubulures suivant le sens de circulation sortant, le guide en T s'étendant entre la ou les deux prises d'entrée pour former le segment de pompage d'irrigation ou d'aspiration de part et d'autre du logement entre chaque prise d'entrée et la tête du T.

Le guide en T permet de protéger la ou les deux tubulures dans les deux sens de circulation entrant et sortant sans qu'il soit nécessaire de prévoir un couvercle au support. Le logement guide la ou les deux tubulures, en permettant de les superposer l'une par rapport à l'autre pour réduire l'encombrement de la cassette par comparaison à une disposition de deux tubulures dans un même plan. En s'étendant entre les deux prises d'entrée des tubulures, le guide en T permet de surcroît de former un segment de pompage pour chacune des deux tubulures d'irrigation et d'aspiration.

### Brève description des dessins

D'autres avantages de l'invention apparaîtront à la lecture de la description d'un mode de réalisation illustré ci-après par les dessins.
La figure 1 montre en perspective une cassette destinée à être reçue par la machine selon l'invention.
La figure 2 montre la cassette en vue de face.
La figure 3 montre la cassette suivant une coupe A-A.
La figure 4 montre la casette suivant une coupe B-B.
La figure 5 montre la casette suivant une vue de dessous.
La figure 6 montre la cassette suivant une coupe C-C.
La figure 7 montre la casette suivant une coupe D-D.
La figure 8 montre un autre exemple de réalisation d'une cassette destinée à être reçue par la machine selon l'invention.
La figure 9 montre la cassette de la figure 8 en vue de dessous.
La figure 10 montre la cassette de la figure 8 suivant une coupe F-F.
La figure 11 montre un exemple de réalisation d'une cassette selon la figure 8, dans lequel on a retiré la fonction d'aspiration pour ne laisser que la fonction d'irrigation.
La figure 12 montre la cassette de la figure 11 en vue de dessous.
La figure 13 montre en perspective une machine d'irrigation et d'aspiration selon l'invention dans laquelle est insérée une cassette destinée à être reçue par la machine selon l'invention.
La figure 14 montre en vue de dessus un chariot et un porte-cassette de la machine illustrée par la figure 13.
La figure 15 est une vue de côté de la figure 14.
La figure 16 est une vue selon la coupe H-H de la figure 14.
La figure 17 montre plus particulièrement le chariot de la figure 13.
La figure 18 montre plus particulièrement le porte-cassette de la figure 13.

**Tableau 1**

| | |
|---|---|
| 1i,1a | Tubulure d'irrigation, d'aspiration |
| 3i,3a | Prises d'entrée |
| 5 | Support |
| 7i,7a | Prises de sortie |
| 9i,9a | Coudes |
| 10i,10a | Pièces tubulaires d'irrigation, d'aspiration |
| 11i, 11a | Segment de pompage d'irrigation, d'aspiration |
| 13 | Guide en T |
| 14 | Capot de protection |
| 15i,15a | Extrémités d'entrée |
| 17 | Arrondi |
| 19 | Logement |
| 21 | Boîtier |
| 23i,23a | Canaux d'entrée |
| 25 | Troisième canal d'entrée |
| 26 | Extrémité |
| 27 | Supports en forme de demi-disques |
| 28 | Voie de communication |
| 29i,29a | Canaux de sortie |
| 31 | Chambre |
| 33,35 | Tubulures d'aspiration complémentaires |
| 36 | Paroi de fond |
| 37,39 | Tubulures d'irrigation complémentaires. |
| 41 | Tubulure d'aspiration complémentaire |
| 43 | Chambre |
| 45 | Paroi de fond |
| 47 | Prises de pression |
| 48 | Pièce en élastomère |
| 49 | Lignes de pression |
| 51i,51a | Pompe péristaltique d'irrigation, d'aspiration |
| 53i,53a | Sabots |
| 54i,54a | Roues |
| 55i,55a | Galets |
| 61- | Châssis |
| 62 | Ressorts de compression |
| 63 | Chariot |
| 64 | Butée |
| 65 | Porte-cassette |
| 66 | Capteur de position |
| 67a,69a | Obturateurs |
| 68 | Capteurs de position |
| 71 | Troisième obturateur |
| 73 | Moyen de verrouillage |
| 75 | Butée |
| 77 | Doigts de reconnaissance |
| 79 | Moyen de centrage |
| 83 | Glissières |
| 85 | Actionneur linéaire |
| 86 | Tige |
| 87 | Glissières |
| 88 | Capteurs de position |
| 89 | Glissières |
| 90 | Capteurs de position |
| 91 | Ressorts |
| 93 | Glissières |
| 94 | Actionneurs linéaires |
| 95 | Tétines |
| 96 | Actionneur linéaire |

### Mode(s) de réalisation de l'invention

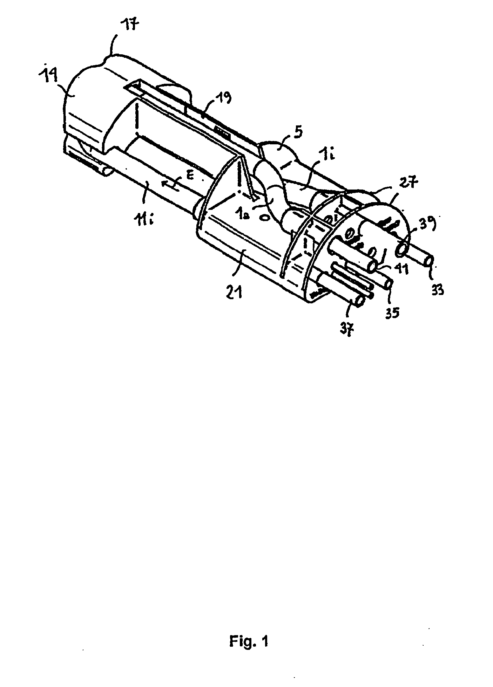

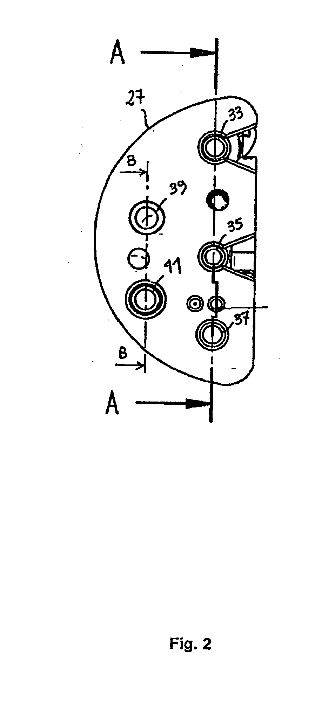

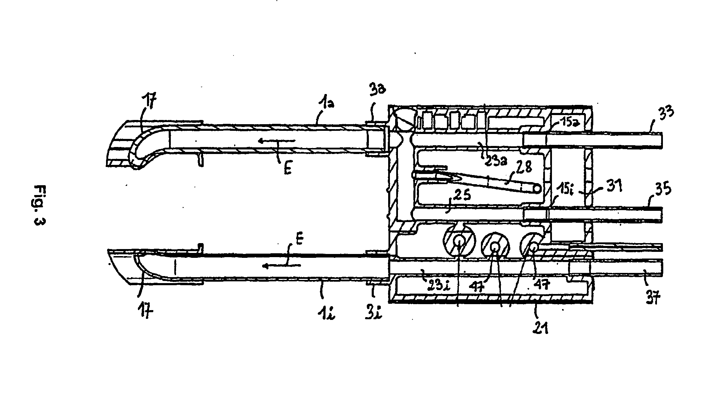

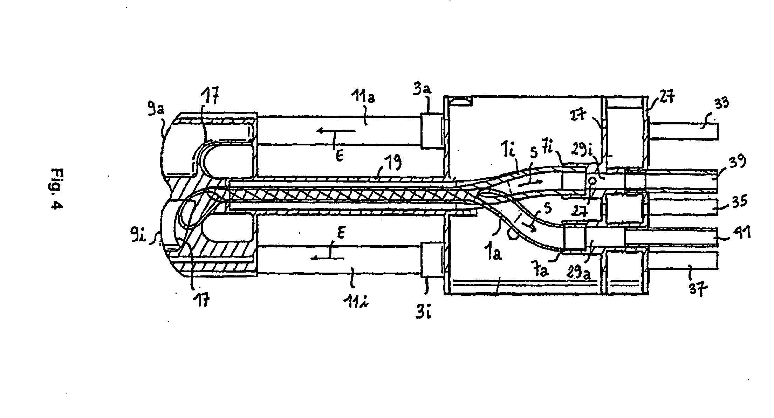

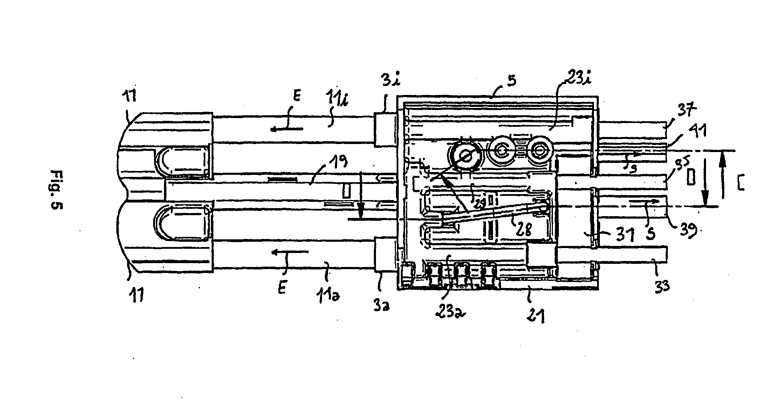

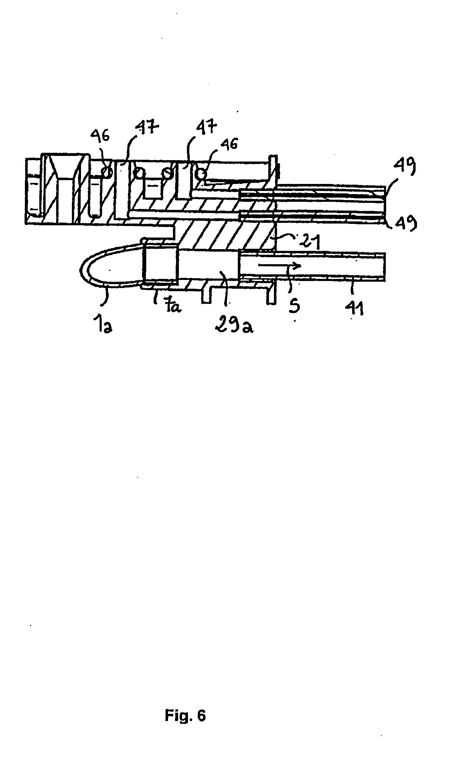

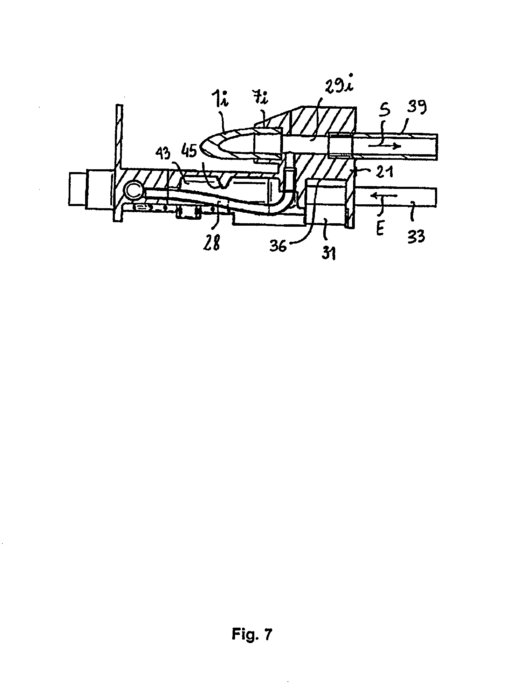

En référence aux figures 1 à 7, une cassette destinée à s'insérer dans une machine d'irrigation et d'aspiration utilisée en endoscopie comprend une tubulure d'irrigation 1i, une tubulure d'aspiration 1a et un support 5 muni de deux prises d'entrée 3i,3a et de deux prises de sortie 7i,7a. Les deux tubulures forment l'une et l'autre un coude 9i,9a pour s'engager avec les deux prises d'entrée et de sortie suivant un sens de circulation respectivement entrant E et sortant S et forment l'une ou l'autre un segment de pompage d'irrigation 11i ou d'aspiration 11 a suivant le sens entrant E de circulation.

Selon l'invention, le support 5 comprend un guide en T 13 conformé à la tête du T pour protéger le coude 9i,9a de chaque tubulure 1 i,1a et conformé le long du corps du T en un logement 19 guidant les deux tubulures superposées l'une 1i par rapport à l'autre 1a suivant le sens de circulation sortant S.

Dans l'exemple illustré par les figures 1 à 7, la tête du T comprend un capot de protection 14 et forme un double arrondi 17 pour guider les deux coudes 9i,9a de chaque tubulure.

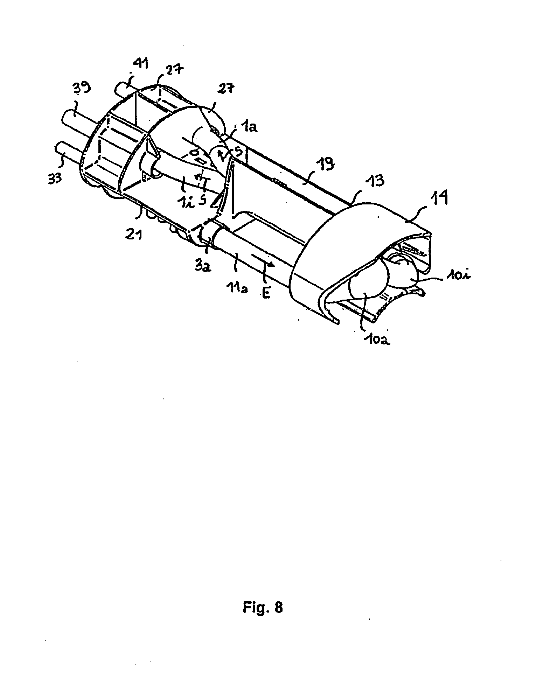

Dans l'exemple de la figure 8, la tête du T comprend là encore un capot de protection 14 mais les coudes 9i,9a sont formés par des pièces tubulaires 10i,10a fabriquées dans une matière plastique souple et insérées entre deux portions de chaque tubulure d'irrigation et d'aspiration.

Le guide en T 13 s'étend entre les deux prises d'entrée 3i,3a pour former le segment de pompage d'irrigation 11 i ou d'aspiration 11 a de part et d'autre du logement 19 entre chaque prise d'entrée 3i,3a et la tête du T.

Le guide en T 13 est fixé à un boîtier 21 intégré au support 5 et pourvu de deux canaux d'entrée 23i,23a ouverts à une extrémité d'entrée 15i,15a et débouchant à une extrémité opposée par les prises d'entrée 3i,3a pour assurer une communication avec les deux tubulures 1i, 1a suivant le sens de circulation entrant E.

Le boîtier 21 est pourvu d'un troisième canal d'entrée 25 ouvert à une extrémité 26 et disposé en dérivation par rapport au canal d'entrée 23a communiquant avec la tubulure d'aspiration 1 a pour déboucher, à une extrémité opposée, par la prise d'entrée 3a assurant la communication avec la tubulure d'aspiration 1a.

Le canal d'entrée 23a communiquant avec la tubulure d'aspiration 1 a et le troisième canal d'entrée 25 monté en dérivation s'ouvrent, à l'extrémité opposée à la prise d'entrée 3a assurant la communication avec la tubulure d'aspiration 1a, dans une chambre 31 intégrée au boîtier et recevant deux tubulures d'aspiration complémentaires 33,35 s'engageant avec ces deux canaux 23a,25 en étant disposées à distance d'une paroi de fond 36 de la chambre 31 pour être comprimées contré cette paroi de fond 36 dans une position d'obstruction de ce canal d'entrée 23a communiquant avec la tubulure d'aspiration 1a ou de ce troisième canal d'entrée 25.

Le boîtier 21 est pourvu de deux canaux de sortie 29i,29a ouverts à une extrémité de sortie et débouchant à une extrémité opposée par les prises de sortie 7i,7a pour assurer une communication avec les deux tubulures 1i, 1a suivant le sens de circulation sortant S.

Les canaux de sortie 29i,29a sont portés par des supports en forme de demi-disques 27 s'étendant dans un plan-perpendiculaire à un plan du boîtier pour être surélevés par rapport aux canaux d'entrée 23i,23a,25.

Le boîtier 21 est pourvu d'une voie de communication 28 entre le canal de sortie 29i communiquant avec la tubulure d'irrigation 1i et le canal d'entrée 23a communiquant avec la tubulure d'aspiration 1 a ou le troisième canal d'entrée 25 monté en dérivation par rapport à ce dernier.

La voie de communication 28 est assurée par une tubulure disposée dans une chambre 43 intégrée au boîtier 21 et à distance d'une paroi de fond 45 de cette chambre pour être comprimée contre cette paroi de fond dans une position d'obstruction de cette voie de communication.

Dans l'exemple illustré par les figures 1 à 7, le boîtier 21 incorpore une ou deux prises de pression 47 formées de conduits pour le passage d'air provenant de lignes de pression 49 branchées sur une tubulure d'irrigation complémentaire 39 par l'intermédiaire d'un détecteur de pression à membrane.

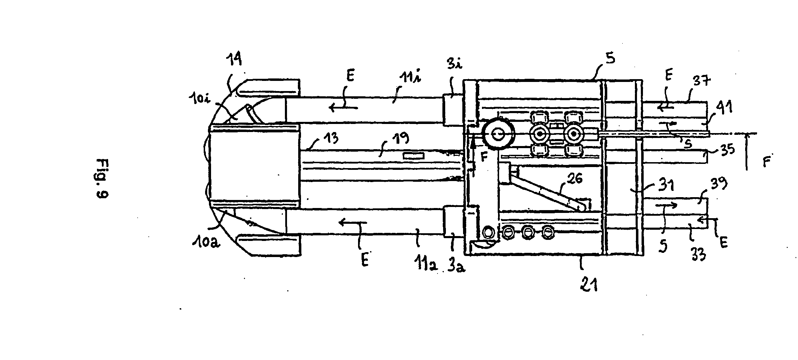

Dans l'exemple illustré par la figure 9, le boîtier 21 est prévu pour recevoir une pièce en élastomère 48 formant d'une seule pièce, les deux prises de pression 47 et les conduits pour le passage d'air provenant des lignes de pression 49.

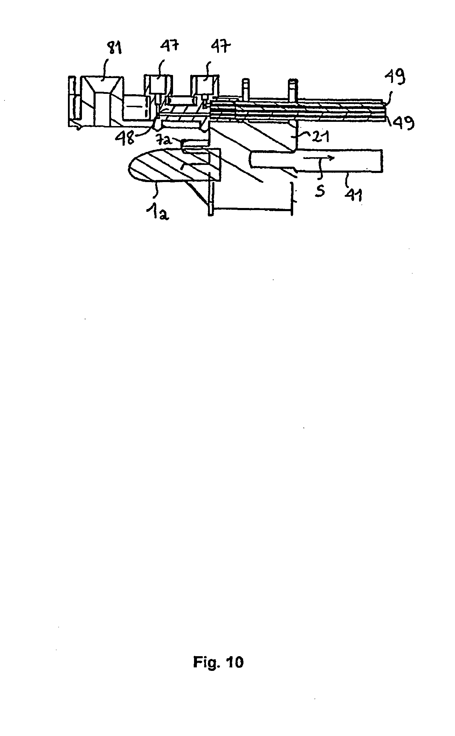

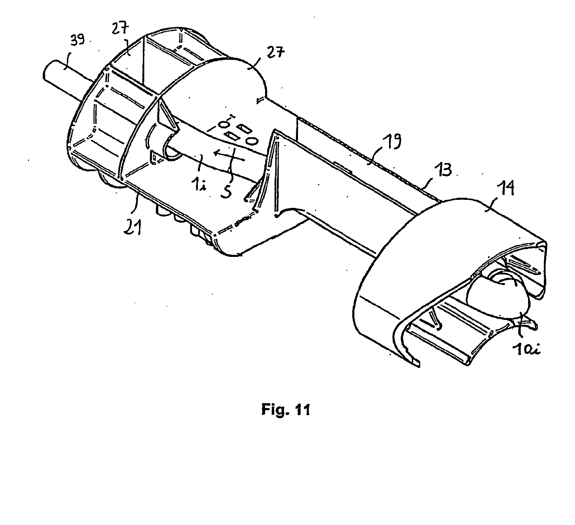

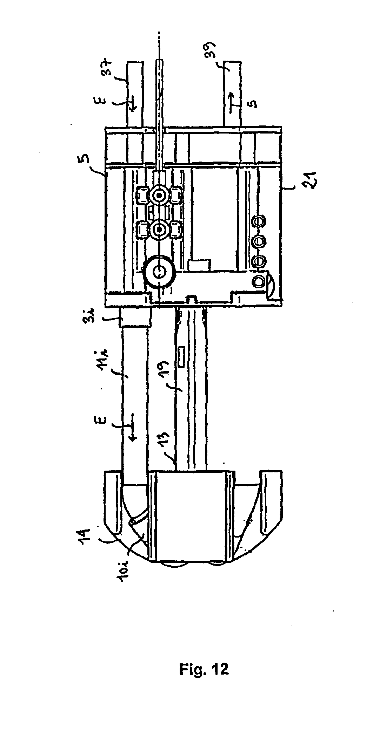

Dans l'exemple illustré par les figures 11 et 12, ont été retirés par rapport à l'exemple de réalisation illustré par les figures 8 à 10, la tubulure d'aspiration 1a, la prise d'entrée 3a et le segment de pompage d'aspiration 11a. Dans le boîtier 21, ont été également retirés le canal d'aspiration 23a, le canal de sortie 29a ainsi que les tubulures d'aspiration complémentaires 33,35 et 41. Dans cet exemple de réalisation, la cassette ne permet de réaliser que l'irrigation et convient plus particulièrement pour une utilisation en endoscopie à des fins de diagnostic.

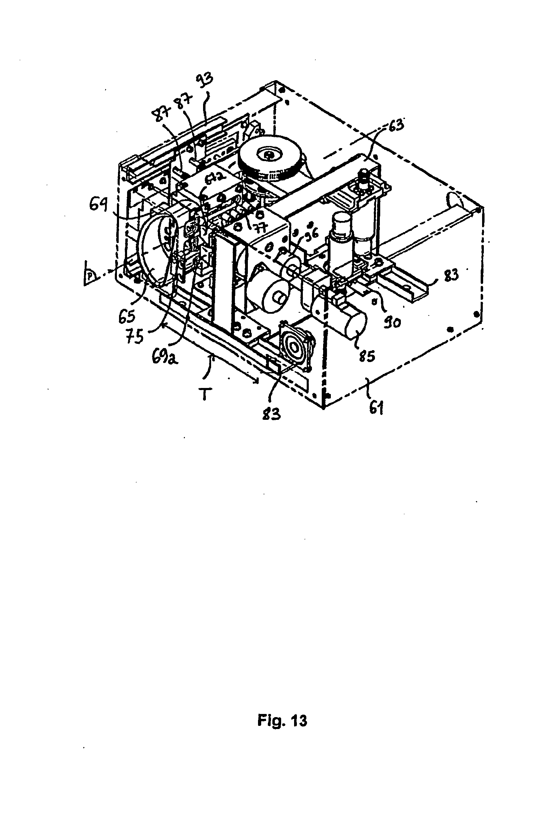

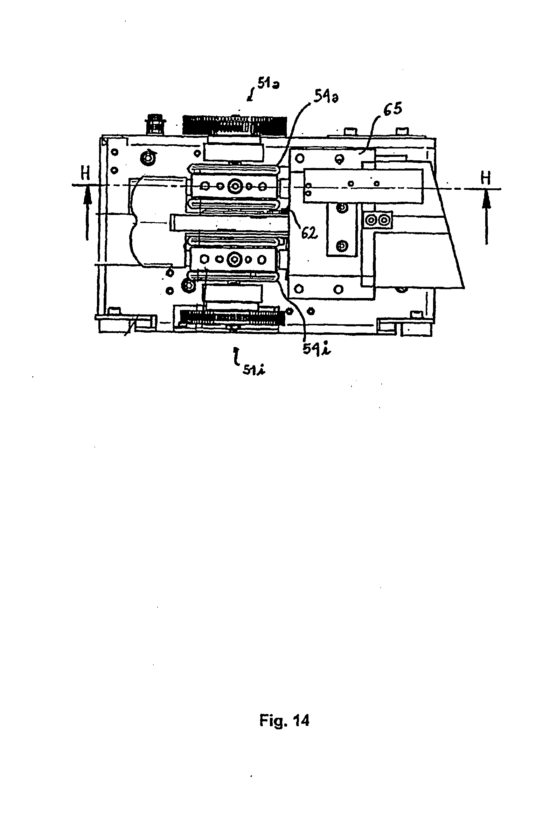

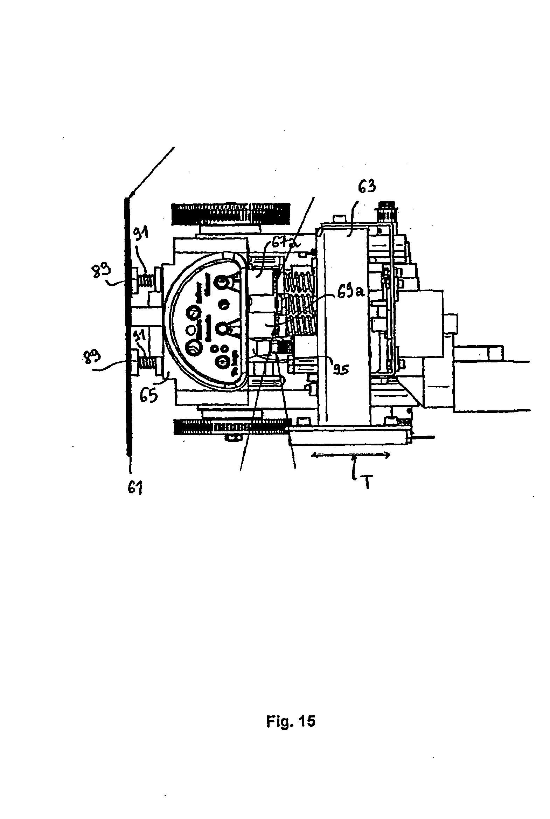

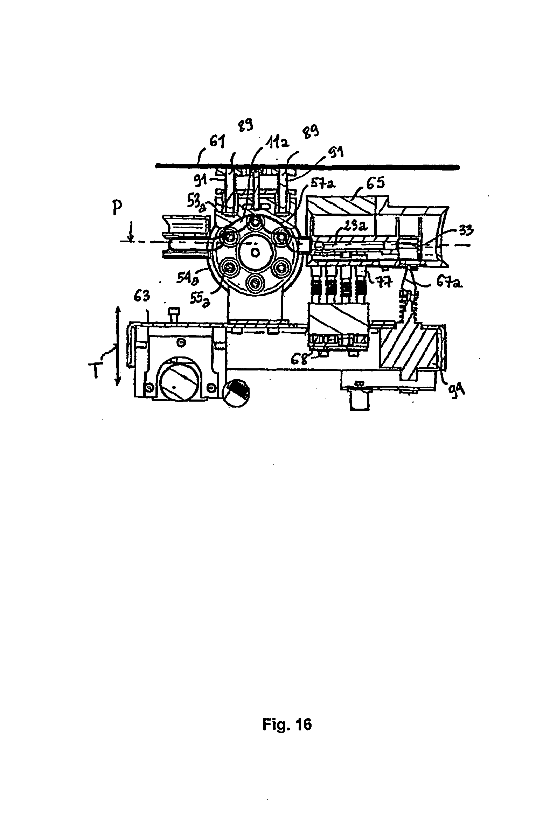

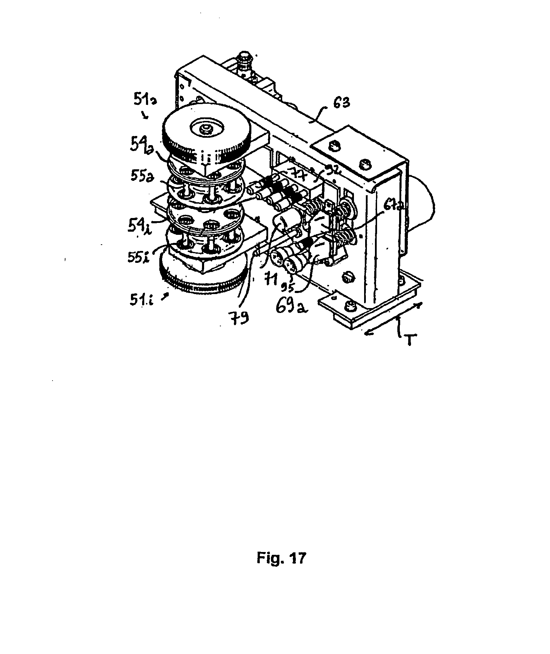

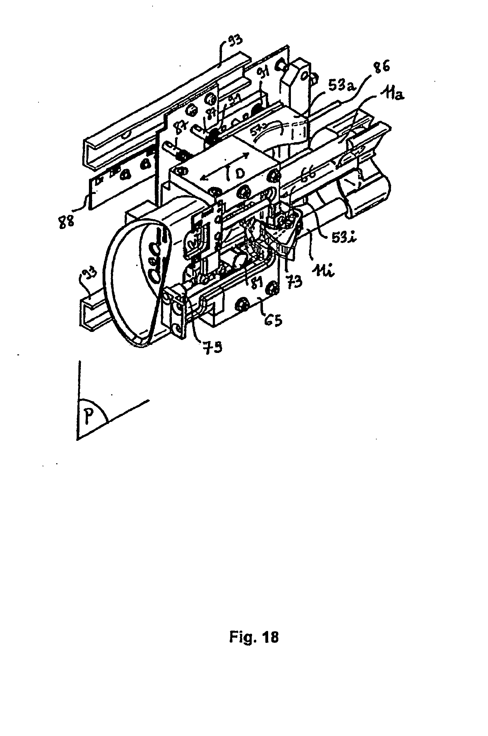

En référence aux figures 13 à 18, la machine selon l'invention comprend une pompe péristaltique d'irrigation 51 i à sabot 53i et à roue 54i à galets 55i montés en correspondance, l'un 53i sur un châssis 61 et l'autre 54i sur un chariot 63 mobile le long de glissières 83 fixées au châssis 61 pour s'étendre suivant une direction de translation T. Un actionneur linéaire 85 commande la translation du chariot 63 entre une position de repos par desserrement du sabot 53i par rapport aux galets 55i et une position de pompage par resserrement du sabot 53i par rapport aux galets 55i. La machine comprend également un porte-cassette 65 monté sur le châssis 61 pour s'étendre dans un plan P perpendiculaire à la direction de translation T et passant entre le sabot 53i et la roue 54i à galets 55i de la pompe d'irrigation 51 i.

Selon l'invention, la machine comprend une pompe péristaltique d'aspiration 51a à sabot 53a et à roue 54a à galets 55a montés en correspondance, l'un 53a sur le châssis 61 et l'autre 54a sur le chariot 63 pour desserrer ou resserrer ledit sabot 53a par rapport audits galets 55a suivant la direction de translation T lors du desserrement ou du resserrement du sabot 53i par rapport aux galets 55i de la pompe péristaltique d'irrigation 51i entre la position de repos et la position de pompage, le plan P perpendiculaire à la direction de translation T passant également entre le sabot 53a et la roue 54a à galets 55a de la pompe d'aspiration.

L'agencement du porte-cassette 65 dans un plan perpendiculaire à la direction de translation T du chariot mobile 63 permet de disposer à la fois le segment de pompage d'irrigation 11i et le segment de pompage d'aspiration 11 a d'une cassette entre les sabots 53i, 53a et les galets 55i,55a respectivement de la pompe péristaltique d'irrigation 51i et la pompe péristaltique d'aspiration 51a. La cassette est introduite dans le porte-cassette 65 lorsque les sabots sont desserrés par rapport aux galets dans la position de repos pour que les segments de pompage d'irrigation 11i et d'aspiration 11a soient ensuite laminés entre les sabots et les galets dans la position de pompage.

De préférence, le porte-cassette 65 est monté mobile par rapport au châssis 61 pour être entraîné en déplacement par le chariot 63 lorsque ce dernier est déplacé de la position de repos à la position de pompage. Le déplacement du porte-cassette 65 s'effectue le long de glissières 87 fixées au châssis 61 parallèlement à la direction de translation T. Avantageusement, les sabots 53i,53a des pompes péristaltiques d'irrigation 51i et d'aspiration 51 a sont montés mobiles par rapport au châssis 65 le long de glissières 89 s'étendant parallèlement à la direction de translation T. Le déplacement des sabots 53i,53a s'effectue contre la compression de ressorts 91 disposés autour des glissières 89 pour régler une pression de laminage entre les sabots 53i,53a et les galets 55i,55a lorsque le chariot 63 s'est déplacé dans la position de pompage.

Pour faciliter l'introduction ou le retrait d'une cassette dans la machine d'irrigation et d'aspiration, le porte-cassette 65 est monté mobile le long de glissières 93 fixées au châssis 61 pour être déplacé parallèlement au plan P perpendiculaire à la direction de translation T. Le déplacement du porte-cassette 65 est commandé par un actionneur linéaire entre une position d'insertion de cassette où le porte-cassette 65 s'est rapproché des sabots et des roues à galets des pompes d'irrigation et d'aspiration et une position d'éjection de casette où le porte-cassette 65 s'est éloigné desdits sabots et desdites roues à galets.

La machine d'irrigation et d'aspiration à cassette comprend en outre un moyen de centrage 79 monté sur le chariot 63 pour être déplacé avec le chariot 63 suivant la direction de translation T depuis la position de repos vers la position de pompage et après que le porte-cassette 65 se soit rapproché des sabots 53i,53a et des roues 54i,54a à galets des deux pompes d'irrigation et d'aspiration dans la position d'insertion de cassette. Application industrielle

Le support 5 d'une cassette ainsi que le guide en T 13 avec le capot de protection 14 à la tête du T et le logement 21 le long du corps du T, le boîtier intégré au support 21 pourvu des canaux d'entrée 23i,23a, le troisième canal d'entrée 25, les prises d'entrée 3i,3a, les prises de sortie 7i,7a et les supports 27 sont d'une seule pièce de préférence modulée par injection de matière plastique.

L'utilisation de la cassette avec la machine d'irrigation et d'aspiration selon l'invention s'effectue de la façon suivante. La cassette est introduite à la main dans le porte-cassette 65 jusqu'à ce qu'un moyen de verrouillage 73 monté en pivot par rapport au porte-cassette 65 vienne verrouiller le support 5 de la cassette par rapport au porte-cassette 65 contre la compression de ressorts 62 portés par le porte-cassette. Le pivotement du moyen de verrouillage 73 est détecté par un capteur de position 66 fixé au porte-cassette 65.

Il convient de noter que le capot de protection 14 et les supports en forme de demi-disques 27 offrent une fonction pour détromper un utilisateur lors de l'insertion de la cassette dans le porte-cassette 65.

L'actionneur linéaire commandant le déplacement du porte-cassette 65 déplace ce dernier dans la position d'insertion de cassette pour disposer les segments de pompage d'irrigation 11 i et d'aspiration 11a des tubulures d'irrigation 1i et d'aspiration 1a entre les sabots 53i,53a et les roues 54i,54a à galets respectivement de la pompe d'irrigation et de la pompe d'aspiration. Des capteurs de position 88 sont fixés au châssis 61 pour contrôler le déplacement du porte-cassette 65 dans le plan P perpendiculaire à la direction de translation T. Une tige 86 est fixée à l'actionneur linéaire commandant le déplacement du porte-cassette 65 pour venir en butée contre le châssis arrêter le déplacement du porte-cassette dans la position d'insertion de la cassette.

L'actionneur linéaire 85 commandant le déplacement du chariot mobile 63 déplace ensuite ce dernier suivant la direction de translation T de la position de repos à la position de pompage pour entraîner en déplacement le porte-cassette 65 et venir presser les segments de pompage d'irrigation 1i et d'aspiration 1 a de la cassette entre les sabots et les galets respectivement de la pompe d'irrigation et de la pompe d'aspiration. Des capteurs de position 90 sont fixés au châssis 61 pour contrôler le déplacement du chariot 63 suivant la direction de translation T. Les sabots 53i,53a des pompes péristaltiques d'irrigation et d'aspiration se déplacent contre la compression des ressorts 91 disposés autour des glissières 87 pour régler une pression de laminage entre les sabots 53i,53a et les galets 55i,55a lorsque le chariot 63 s'est déplacé dans la position de pompage. Le moyens de centrage 79 porté par le chariot mobile 63 s'insère dans un moyen de centrage 81 porté par le boîtier 21 de la cassette pour centrer la cassette par rapport au chariot 63 dans la position de pompage.

Avantageusement, le chariot 63 porte des doigts de reconnaissance de cassette 77 mobiles par rapport au chariot suivant la direction de translation T. Les doigts de reconnaissance de cassette 77 sont déplacés par le chariot parallèlement à la direction de translation T pour coopérer avec des repères correspondants formés dans le boîtier 21 de la cassette pour reconnaître cette dernière dans la position de pompage et ainsi prérégler certains paramètres de fonctionnement de la machine d'irrigation et d'aspiration, notamment la vitesse de rotation de la roue à galets de la pompe d'irrigation. Des capteurs de position 68 sont prévus sur le châssis 63 pour détecter la présence ou l'absence des doigts de reconnaissance de cassette 77 et ainsi identifier la cassette insérée dans la machine d'irrigation et d'aspiration.

La tubulure d'irrigation 1i est alimentée en fluide physiologique à partir d'un réservoir et d'une tubulure d'irrigation complémentaire 37 s'engageant dans le canal d'entrée 3i du boîtier 21. Le segment de pompage 11i de la tubulure d'irrigation 1i coopère avec la pompe péristaltique d'irrigation 51 i disposée dans la machine d'irrigation et d'aspiration dans laquelle la cassette est insérée pour mettre en circulation le fluide physiologique dans la tubulure d'irrigation complémentaire 39 s'engageant dans le canal de sortie 29i communiquant avec la tubulure d'irrigation 1 i et débouchant dans une canule d'endoscope placée dans une zone d'intervention chirurgicale d'un patient, par exemple une articulation du genou ou de l'épaule.

Le segment de pompage 11a de la tubulure d'aspiration 1a coopère avec la pompe péristaltique d'aspiration 51 a disposée dans la machine d'irrigation et d'aspiration pour aspirer, dans la tubulure d'aspiration 1a, un fluide provenant soit d'une canule soit d'un autre outil de chirurgie, par exemple un « shaver », par l'intermédiaire respectivement des tubulures d'aspiration complémentaires 33 et 35, l'une étant comprimée pour être obstruée lorsque l'autre est en service. A cet effet, le chariot 63 porte deux obturateurs 67a,69a mobiles par rapport au chariot 63 suivant la direction de translation T. Des actionneurs linéaires 94 commandent en déplacement l'un ou l'autre des obturateurs pour comprimer l'une ou l'autre des tubulures d'aspiration complémentaires 33,35 contre la paroi de fond 37 de la chambre 31 intégrée au boîtier 21 de la cassette dans la position d'obstruction du canal d'entrée 23a communiquant avec la tubulure d'aspiration 1a ou dans la position d'obstruction du troisième canal d'entrée 25 monté en dérivation par rapport à ce canal d'entrée 23a.

Le fluide aspiré circule vers un réceptacle extérieur à la machine d'irrigation et d'aspiration par l'intermédiaire d'une tubulure d'aspiration complémentaire 41 s'engageant dans le canal de sortie 29a communiquant avec la tubulure d'aspiration 1a.

La voie de communication 28 entre le canal de sortie 29i communiquant avec la tubulure d'irrigation 1i et le canal d'entrée 23a communiquant avec la tubulure d'aspiration 1a ou le troisième canal d'entrée 25 monté en dérivation par rapport à ce dernier est commandée d'une position d'obstruction à une position de circulation pour gérer une surpression accidentelle dans l'articulation du patient. A cet effet, le chariot mobile 63 porte un troisième obturateur 71 mobile par rapport au chariot 63 suivant la direction de translation T. Un actionneur linéaire 96 commande en déplacement le troisième obturateur pour comprimer la tubulure de communication 28 contre la paroi de fond 45 de la chambre 43 intégrée au boîtier 21 dans la position d'obstruction de cette tubulure de communication 28.

Avantageusement, le chariot mobile 63 porte une ou deux prises de pression d'air formées de tétines 95 communiquant avec des capteurs de pression fixés par exemple au châssis 61 pour déterminer la pression détectée par exemple sur la tubulure complémentaire d'irrigation 39 par un détecteur à membrane. Les tétines 95 sont disposées sur le chariot mobile 63 pour s'insérer dans les prises de pression 47 incorporées au boîtier 21 de la cassette selon l'invention lorsque le chariot mobile 63 s'est déplacé dans la position de pompage. Un joint 46 est monté autour des prises de pression 47 incorporées au boîtier pour assurer un contact étanche à l'air avec les tétines dans la position de pompage du chariot mobile 63.

En fin d'utilisation, l'actionneur linéaire 85 commandant le déplacement du chariot mobile 63 déplace ce dernier suivant la direction de translation T de la position de pompage à la position de repos pour desserrer les segments de pompage d'irrigation 1i et d'aspiration 1a entre les sabots et les galets respectivement de la pompe d'irrigation et de la pompe d'aspiration. Les capteurs de position 90 fixés au châssis 61 contrôlent le déplacement du chariot 63 suivant la direction de translation T. Une butée est fixée au châssis 61 pour arrêter le déplacement du chariot dans la position de repos.

L'actionneur linéaire commandant le déplacement du porte-cassette 65 déplace ensuite ce dernier dans la position d'éjection de cassette pour éloigner les segments de pompage d'irrigation 11i et d'aspiration 11a des sabots 53i,53a et des roues 54i,54a à galets respectivement de la pompe d'irrigation et de la pompe d'aspiration. Les capteurs de position 88 fixés au châssis 61 contrôlent le déplacement du porte-cassette 65 dans le plan P perpendiculaire à la direction de translation T. Une butée 64 est fixée au châssis pour arrêter le déplacement du porte-cassette dans la position d'éjection de cassette.

Pour retirer à la main la cassette de la machine d'irrigation et d'aspiration, le moyen de verrouillage 73 monté en pivot par rapport au porte-cassette 65 est actionné par une butée 75 fixée au châssis 63 pour pivoter par rapport au porte-cassette 65 lorsque ce dernier se déplace parallèlement au plan P perpendiculaire à la direction de translation T, de la position d'insertion de cassette à la position d'éjection de cassette. Les ressorts de compression 62 portés par le porte-cassette éjectent alors la cassette du porte-cassette. La machine d'irrigation et d'aspiration est prête à être utilisée avec une nouvelle cassette.

## Revendications

1. Machine d'irrigation et d'aspiration utilisée en endoscopie et destinée à recevoir une cassette comprenant une tubulure d'irrigation (1 i) ou une tubulure d'aspiration (la) et un support (5) muni d'une (3i) ou de deux prises d'entrée (3i, 3a) et d'une (7i) ou de deux prises de sortie (7i, 7a), la ou les deux tubulures formant un coude (9i, 9a) pour s'engager avec la ou les deux prises d'entrée et de sortie suivant un sens de circulation respectivement entrant (E) et sortant (S) et formant un segment de pompage d'irrigation (11i) ou d'aspiration (11 a) suivant le sens entrant (E) de circulation, le support comprenant un guide en T conformé à la tête du T pour protéger le coude de chaque tubulure et conformé le long du corps du T en un logement guidant la ou les deux tubulures suivant le sens de circulation sortant, le guide en T s'étendant entre la ou les deux prises d'entrée pour former le segment de pompage d'irrigation ou d'aspiration de part et d'autre du logement entre chaque prise d'entrée et la tête du T, ladite machine comprenant une pompe péristaltique d'irrigation (51 i) à sabot (53i) et à roue (54i) à galets (55i) montés en correspondance, l'un (53i) sur un châssis (61) et l'autre (54i) sur un chariot (63) mobile par rapport au châssis suivant une direction de translation (T) entre une position de repos où le sabot (53i) est desserré par rapport aux galets (55i) et une position de pompage où le sabot (53i) est resserré par rapport aux galets (55i) et un porte-cassette (65) monté sur le châssis (61) pour s'étendre dans un plan (P) perpendiculaire à la direction de translation (T) et passant entre le sabot (53i) et la roue (54i) à galets (55i) de la pompe d'irrigation, et comprenant une pompe péristaltique d'aspiration (51 a) à sabot (53a) et à roue (54a) à galets (55a) montés en correspondance, l'un (53a) sur le châssis (61) et l'autre (54a) sur le chariot (63) pour desserrer ou resserrer ledit sabot (53a) par rapport audits galets (55a) suivant la direction de translation (T) lors du desserrement ou du resserrement du sabot (53i) par rapport aux galets (55i) de la pompe péristaltique d'irrigation (51 i) dans la position de repos ou la position de pompage, le plan (P) dans lequel le porte-cassette (65) s'étend passant également entre le sabot (53a) et la roue (54a) à galets (55a) de la pompe péristaltique d'aspiration et ladite machine étant **caractérisée en ce que** :
- le porte-cassette (65) est monté mobile par rapport au châssis (61) suivant la direction de translation (T) pour être entraîné en translation par le chariot (63) lorsque ce dernier est déplacé de la position de repos à la position de pompage et
- le porte-cassette (65) est monté mobile par rapport au châssis (61) pour être déplacé parallèlement au plan (P) perpendiculaire à la direction de translation (T), entre une position d'insertion de cassette où le porte-cassette (65) s'est rapproché des sabots (53i, 53a) et des roues (54i, 54a) à galets des pompes péristaltiques d'irrigation (51 i) et d'aspiration (51 a) et une position d'éjection de cassette où le porte-cassette (65) s'est éloigné desdits sabots et desdites roues à galets, définies respectivement par une première (86) et une deuxième butées (64) par rapport au châssis (61).

2. Machine d'irrigation et d'aspiration selon la revendication 1, **caractérisée en ce que** le porte-cassette (65) est pourvu d'un moyen de verrouillage (73) monté en pivot par rapport au porte-cassette (65) pour être actionné par une butée (75) fixée au châssis (63) et pivoter par rapport au porte-cassette (65) lorsque ce dernier se déplace parallèlement au plan (P) perpendiculaire à la direction de translation (T), de la position d'insertion de cassette à la position d'éjection de cassette.

3. Machine d'irrigation et d'aspiration selon la revendication 1 ou 2, **caractérisée en ce que** les sabots (53i, 53a) des pompes péristaltiques d'irrigation (51 i) et d'aspiration (51 a) sont montés mobiles par rapport au châssis (65) suivant la direction de translation (T).

4. Machine d'irrigation et d'aspiration selon la revendication 1 ou 2, **caractérisée en ce qu'**elle comprend des capteurs de pression d'air communiquant avec des prises de pression d'air (95) portées par le chariot mobile (63).

5. Machine d'irrigation et d'aspiration selon la revendication 1 ou 2, **caractérisée en ce que** le chariot (63) porte deux (67a, 69a) ou trois (71) obturateurs mobiles par rapport au chariot (63) suivant la direction de translation (T).

6. Machine d'irrigation et d'aspiration selon la revendication 1 ou 2, **caractérisée en ce que** le chariot (63) porte des doigts de reconnaissance de cassette (77) mobiles par rapport au chariot suivant la direction de translation (T).

7. Machine d'irrigation et d'aspiration selon la revendication 1 ou 2, **caractérisée en ce qu'**elle comprend un moyen de centrage (79) monté sur le chariot (63) pour être déplacé avec le chariot (63) suivant la direction de translation (T) depuis la position de repos vers la position de pompage et après que le porte-cassette se soit rapproché des sabots (53i, 53a) des deux pompes d'irrigation et d'aspiration dans la position d'insertion de cassette.

## Patentansprüche

1. Spül- und Saugmaschine, die bei der Endoskopie einsetzbar und dazu eingerichtet ist, eine Kassette aufzunehmen, die über ein Spülrohr (1i) oder ein Saugrohr (Ia) und über einen Träger (5) verfügt, der mit einem (3i) oder mit zwei Einlassanschlüssen (3i, 3a) und mit einem (7i) oder mit zwei Auslassanschlüssen (7i, 7a) ausgestattet ist, wobei das oder die beiden Rohre ein Winkelstück (9i, 9a) ausbilden, um mit dem oder den beiden Einlass- und Auslassanschlüssen in jeweils einer Einlasszirkulationsrichtung (E) und einer Auslasszirkulationsrichtung (S) verbunden zu sein und ein Pumpensegment zum Spülen (11 i) oder zum Absaugen (11 a) entsprechend der Einlasszirkulationsrichtung (E) zu bilden, wobei der Träger über eine T-artige Führung verfügt, die an dem Kopf des T zum Schutz des Winkelstücks jedes Rohres ausgebildet ist und entlang des Körpers des T mit einer Aufnahme zum Führen des oder der beiden Rohre entsprechend der Auslasszirkulationsrichtung ausgebildet ist, wobei sich die T-artige Führung zwischen dem oder den beiden Einlassanschlüssen erstreckt, um das Pumpsegment zum Spülen oder zum Absaugen auf beiden Seiten der Aufnahme zwischen jedem Einlassanschluss und dem Kopf des T zu bilden, wobei die Maschine über eine peristaltische Spülpumpe (51 i) mit einem Schuhstück (53i) und mit einem mit Rollen (55i) versehenen Rad (54i) verfügt, die einander zugeordnet angebracht sind, wobei eines (53i) an einem Gehäuse (61) und das andere (54i) an einem Schlitten (63) angebracht sind, der in Bezug auf das Gehäuse entlang einer Translationsrichtung (T) zwischen einer Ruhestellung, in der das Schuhstück (53i) in Bezug auf die Rollen (55i) gelöst ist, und einer Pumpstellung, in der das Schuhstück (53i) in Bezug auf die Rollen (50i) festgelegt ist, beweglich ist, und mit einem Kassettenträger (65), der an dem Gehäuse (61) derart angebracht ist, dass er sich in einer Ebene (P) rechtwinklig zu der Translationsrichtung (T) erstreckt und zwischen dem Schuhstück (53i) und dem mit Rollen (55i) versehenen Rad (54i) der Spülpumpe verläuft, und die über eine peristaltische Saugpumpe (51 a) mit einem Schuhstück (53a) und mit einem mit Rollen (55a) versehenen Rad (54a) verfügt, die einander zugeordnet befestigt sind, und zwar das eine (53a) an dem Gehäuse (61) und das andere (54a) an dem Schlitten (63), um das Schuhstück (53a) in Bezug auf die Rollen (55a) in der Translationsrichtung (T) während des Lösens oder Festlegens des Schuhstücks (53i) in Bezug auf die Rollen (50i) der peristaltischen Spülpumpe (51 i) in der Ruhestellung oder in der Pumpstellung zu lösen oder festzulegen, wobei die Ebene (P), in der sich der Kassettenträger (65) erstreckt, weiterhin zwischen dem Schuhstück (53a) und dem mit Rollen (55a) versehenen Rad (54a) der peristaltischen Saugpumpe verläuft, und wobei die Maschine **dadurch gekennzeichnet ist, dass**
- der Kassettenträger (65) in Bezug auf das Gehäuse (61) in der Translationsrichtung (T) beweglich angebracht ist, um über den Schlitten (63) translatorisch angetrieben zu sein, während sich dieser zwischen der Ruhestellung und der Pumpstellung bewegt, und
- der Kassettenträger (65) in Bezug auf das Gehäuse (61) beweglich befestigt ist, um parallel zu der rechtwinklig zu der Translationsrichtung (T) ausgerichteten Ebene (P) zwischen einer Kassetteneinfügestellung, in der der Kassettenträger (65) an die Schuhstücke (53i, 53a) und die mit Rollen versehenen Räder (54i, 54a) der peristaltischen Spül- (51 i) und Saugpumpen (51 a) angenähert ist, und einer Kassettenausstoßstellung, bei der der Kassettenträger (65) von den Schuhstücken und den mit Rollen versehenen Rädern entfernt angeordnet ist, beweglich zu sein, und zwar jeweils festgelegt durch erste (86) und zweite (64) Anschläge in Bezug auf das Gehäuse (61).

2. Spül- und Saugmaschine nach Anspruch 1, bei der der Kassettenträger (65) mit einem Verriegelungsmittel (73) ausgestattet ist, das in Bezug auf den Kassettenträger (65) schwenkbar befestigt ist, um durch einen Anschlag (75) betätigbar zu sein, der an dem Gehäuse (63) angebracht ist, um schwenkbar in Bezug auf den Kassettenträger (65) zu sein, während sich dieser parallel zu der rechtwinklig zu der Translationsrichtung (T) ausgerichteten Ebene (P) von der Kassetteneinfügeposition und der Kassettenauswerfposition bewegt.

3. Spül- und Saugmaschine nach Anspruch 1 oder 2, **dadurch gekennzeichnet, dass** die Schuhstücke (53i, 53a) der peristaltischen Spül- (51 i) und Saugpumpen (51 a) in Bezug auf das Gehäuse (65) entlang der Translationsrichtung (T) beweglich angebracht sind.

4. Spül- und Saugmaschine nach Anspruch 1 oder 2, **dadurch gekennzeichnet, dass** sie über Druckluftfühler verfügt, die mit Druckluftanschlüssen (95) in Verbindung stehen, welche durch den beweglichen Schlitten (63) erhalten sind.

5. Spül- und Saugmaschine nach Anspruch 1 oder 2, **dadurch gekennzeichnet, dass** der Schlitten (63) über zwei (67a, 69a) oder drei (71) Verschlüsse verfügt, die in Bezug auf den Schlitten (63) in der Translationsrichtung (T) beweglich sind.

6. Spül- und Saugmaschine nach Anspruch 1 oder 2, **dadurch gekennzeichnet, dass** der Schlitten (63) Kassettenerkennungsfinger (77) aufweist, die in Bezug auf den Schlitten entlang der Translationsrichtung (T) beweglich sind.

7. Spül- und Saugmaschine nach Anspruch 1 oder 2, **dadurch gekennzeichnet, dass** sie ein Zentriermittel (79) aufweist, das an dem Schlitten (63) angebracht ist, um mit dem Schlitten (63) in der Translationsrichtung (T) von der Ruhestellung in die Pumpstellung bewegbar zu sein und nachdem der Kassettenträger in der Kassetteneinführposition an die Schuhstücke (53i, 53a) der beiden Spül- und Saugpumpen angenähert ist.

## Claims

1. The irrigation and aspiration machine used in endoscopy and intended to receive a cassette comprising an irrigation tube (1i) or an aspiration tube (la) and a support (5) furnished with one (3i) or with two inlet plugs (3i, 3a), and with one (7i) or with two outlet plugs (7i, 7a), the tube or the two tubes forming an elbow (9i, 9a) for engaging with the inlet and outlet plug or the two inlet and outlet plugs in a respectively incoming (E) and outgoing (S) direction of flow and forming a segment of irrigation (11i) or of aspiration (11a) pumping in the incoming direction (E) of flow, the support comprising a T guide shaped to the head of the T so as to protect the elbow of each tube and shaped along the body of the T as a slot guiding the tube or the two tubes in the outgoing direction of flow, the T guide running between the inlet plug or the two inlet plugs so as to form the segment of irrigation or of aspiration pumping on either side of the slot between each inlet plug and the head of the T, the said machine comprising a peristaltic irrigation pump (51i) with shoe (53i) and with wheel (54i) with rollers (55i) mounted in correspondence, one (53i) on a chassis (61) and the other (54i) on a carriage (63) moveable along runners with respect to the chassis so as to extend in a direction of translation T between a rest position by unclamping of the shoe (53i) with respect to the runners (55i) and a pumping position by reclamping of the shoe (53i) with respect to the rollers (55i) and a cassette holder (65) mounted on the chassis (61) so as to extend in a plane (P) perpendicular to the direction of translation (T) and passing between the shoe (53i) and the wheel (54i) with rollers (55i) of the irrigation pump (51i), and comprising a peristaltic aspiration pump (51a) with shoe (53a) and with wheels (54a) with rollers (55a) mounted in correspondence, one (53a) on the chassis (61) and the other (54a) on the carriage (63) so as to unclamp or reclamp said shoe (53a) with respect to said rollers (55a) in the direction of translation (T) upon the unclamping or reclamping of the shoe (53i) with respect to the rollers (55i) of the peristaltic irrigation pump (51i) between the rest position and the pumping position, the plane (P)in which the cassette holder (65) extend passing also between the shoe (53a) and the wheel (54a) with rollers (55a) of the peristaltic aspiration pump and the said machine being **characterized in that**:
- the cassette holder (65) is mounted moveably with respect to the chassis (61) in the direction of translation (T) so as to be driven in translation by the carriage (63) when the latter is displaced from the rest position to the pumping position and
- the cassette holder (65) is mounted moveably with respect to the chassis (61) so as to be displaced parallel to the plan (P) perpendicular to the direction of translation (T), between a cassette insertion position where the cassette holder (65) is close to the shoes (53i, 53a) and wheels (54i, 54a) with rollers of the peristaltic irrigation (51i) and aspiration (51a) pumps and a cassette ejection position where the cassette holder (65) is away from said shoes and said wheels with rollers, defined respectively by a first (86) and a second abutment (64)in respect to the chassis (61).

2. The irrigation and aspiration machine according to claim 1, **characterized in that** the cassette holder (65) is provided with a means of locking (73) mounted pivotably with respect to the cassette holder (65) so as to be actuated by an abutment (75) fixed to the chassis (63) and pivot with respect to the cassette holder (65) when the latter displaces parallel to the plane (P) perpendicular to the direction of translation (T), from the cassette insertion position to the cassette ejection position.

3. The irrigation and aspiration machine according to the claim 1 or 2, **characterized in that** the shoes (53i, 53a) of the peristaltic irrigation (51i) and aspiration (51a) pumps are mounted moveably with respect to the chassis (65) in the direction of translation (T).

4. The irrigation and aspiration machine according to the claim 1 or 2, **characterized in that** it comprises air pressure sensors communicating with air pressure plugs (95) carried by the moveable carriage (63).

5. The irrigation and aspiration machine according to the claim 1 or 2, **characterized in that** the carriage (63) carries two (67a, 69a) or three (71) shutters moveable with respect to the carriage (63) in the direction of translation (T).

6. The irrigation and aspiration machine according to the claim 1 or 2, **characterized in that** the carriage (63) carries cassette recognition fingers (77) moveable with respect to the carriage in the direction of translation (T).

7. The irrigation and aspiration machine according to the claim 1 or 2, **characterized in that** it comprises a means of centering (79) mounted on the carriage (63) so as to be displaced with the carriage (63) in the direction of translation (T) from the rest position to the pumping position and after the cassette holder has come near to the shoes (53i, 53a) of the two irrigation and aspiration pumps in the cassette insertion position.
